Europäisches Patentamt

European Patent Office

Office européen des brevets

Publication number: **0 273 469**

**A1**

# EUROPEAN PATENT APPLICATION

Application number: **87202214.0**

Int. Cl.⁴: **A61K 31/575** , **A61K 9/22**

Date of filing: **13.11.87**

The title of the invention has been amended (Guidelines for Examination in the EPO, A-III, 7.3).

Priority: **28.11.86 IT 2251086**

Date of publication of application:
**06.07.88 Bulletin 88/27**

Designated Contracting States:
**AT BE CH DE ES FR GB GR LI LU NL SE**

Applicant: **Gipharmex S.p.A.**
**Via P. Palagi 2**
**I-20129 Milano(IT)**

Inventor: **Frigerio, E. Guiliano**
**Via Vittorio Veneto 107**
**I-20091 Bresso Milan(IT)**
Inventor: **Castagnola, Virginio**
**Via Crimea 23**
**I-20147 Milan(IT)**

Representative: **Appoloni, Romano et al**
**ING. BARZANO' & ZANARDO MILANO S.p.A.**
**Via Borgonuovo 10**
**I-20121 Milano(IT)**

## Oral pharmaceutical compositions with sustained release.

The present invention relates to a group of compounds of steroidal structure, favourably interfering in alterations of metabolism of cholesterol, and prepared in the form of pharmaceutical compositions with controlled and time-delayed release, suitable for being used with extremely simplified administration plans and with considerable advantages at the level of therapeutical activity.

EP 0 273 469 A1

# "PHARMACEUTICAL COMPOSITIONS FOR ORAL USAGE OF THE TYPE WITH RELEASE CONTROLLED OVER TIME, CONTAINING COMPOUNDS OF STEROIDAL TYPE, ACTIVE IN THE ALTERATIONS OF METABOLISM OF CHOLESTEROL"

The alterations in cholesterol metabolism represent an extremely frequent pathological condition in the modern world, not in connexion with genetic factors, which are anyway possible and considerable, but rather, and much more frequently, to alimentary habits which are incorrect from both quality and quantity viewpoint; in fact, the nourishment in the industrialized Countries reserves often a privileged role to the unrestrained intake of food containing a high percentage of proteinic and lipidic components of animal origine, with a large content of saturated fatty acids, whilst the consumption is generally relatively lower of complex carbohydrates, of proteins of vegetable origin, and of fats which, as those of vegetable origin, perform a useful protecting effect towards the arising of alterations of the lipidic metabolism in general, and of the metabolism of cholesterol in particular.

On the other side, it is important to keep in mind that these last two alterations can be the cause of many and different clinical forms, which are sometimes very serious, even endangering the same life of the patient.

It is known, e.g., how hypercholesterolemy represents one of the main risk factors in atherosclerotic disease and in myocardial infarction.

The organs involved in the metabolism of cholesterol are several, and comprise the gastroenteric apparatus in its whole, which takes part to both the processes of absorption and to the processes of excretion following the biliar excretion of the compound; liver, which represents an obliged and basic stage in cholesterol metabolic processes (synthesis, conjugation, transformation, biliar excretion); and, finally, various peripheral tissues, with particular reference to the vessel structures (coronary arteries, cerebral vessels, aorta and so forth), which not only represent a considerable metabolic stage, but, often, also the "target" organs which are more meaningful for the pathologic consequences which can be observed (development of atheromatosis processes and intravascular haemocoagulative alterations).

The liver itself can represent an important "target" organ for cholesterol metabolism alterations, at least as relates to one of the most important products of its secretory activity, viz., bile; in fact, it is known how the metabolic deviation at hepatic level leading to an abnormal biliar secretion of cholesterol, both in absolute and relative (relatively to the secretion of other compounds which favour the dissolving thereof) terms, represents, by itself, a pathologic fact (biliar lithiasic disease), and is the basic condition for the development of cholesterolic biliar calculosis. This latter desease concerns even approximately 15% of the adult population, and it is therefore to be considered as a "social" disease, as the same atherosclerotic disease.

The alterations of cholesterol metabolism have chronic characters and development, and their management necessarily assumes the form of a long-term, or very long-term treatment, sometimes concerning the whole life duration; from a practical viewpoint, the consequence is that the useable drugs must be very well tolerated and their intake by the patient must be as simple as possible.

Cholesterol itself behaves as a key substance in the chain of pathologic events which derive from an altered metabolism thereof; similar chemical structures, with the same steroidal group, and close to it along its metabolic transformation path, can, to a certain extent, compete with it, and enter, with useful outcomes, the altered metabolic chain.

In human chemistry, compounds have been already used, which have a chemical structure similar to cholesterol, and are capable of usefully interfere with the metabolism thereof.

Pharmaceutical forms of such compounds of usual type, i.e., as tablets, or hard-gelatin capsules, have been used.

A first purpose of the present invention is that of optimizing the therapeutical efficacy of preparates of the above described type, rendering the regular assumption of the drug more - schematic, and hence much simpler, with one single daily intake at retiring time; on the other hand, if one considers the metabolsim of cholesterol within the context of the physiologic steps of the biliar secretion, seeing the correlation between the controlled release of the drug during the night time and the most critical step from the viewpoint of biliar supersaturation by cholesterol, which occurs during the nightly fasting time is already much easier; the related experiences demonstrated a "surprising" effect of the drug, which resulted in a "surprising" higher clinic activity.

The present invention intends to achieve further purposes, which can be summarized as follows:

-higher or equal efficacy with smaller dosages than the useful dosages when administered in the conventional forms known from the prior art;

-reduction in treatment costs, a fact which is particularly considerable when the very long treatment time required in these circumstances is taken into consideration;

-decrease of the possible incidence of collateral clinic effects, thanks to the reduction of the generally useful dosages.

In order to achieve such purposes, and other advantages which will result from the following disclosure, the present invention proposes a pharmaceutical composition for oral usage, suitable for the management of the alterations of cholesterol metabolism, characterized in that it contains, as its active principle, a compound selected from the following compounds:

(1) 3α,7α,12α-trihydroxy-5β-cholan-24-oic acid;

(2) 2-[[3α,7α,12α-trihydroxy-24-oxo-5β-cholan-24-yl-]amino ]-ethane-sulphonic-acid;

(3) N-[3α,7α,12α-trihydroxy-24-oxo-cholan-24-yl]amino-acetic acid;

(4) 3α,7β,12α-trihydroxy-5β-cholan-24-oic acid;

(5) 2-[[3α,7β,12α-trihydroxy-24-oxo-5β-cholan-24-yl-]amino]-ethane-sulphonic-acid;

(6) N-[3α,7β,12α-trihydroxy-24-oxo-cholan-24-yl]amino-acetic acid;

(7) 2-[[3α,7α-dihydroxy-24-oxo-5β-cholan-24-yl]amino]-ethane-sulphonic-acid;

(8) N-[3α,7α-dihydroxy-24-oxo-cholan-24-yl]-amino-acetic acid;

(9) 2-[[3α,7β-dihydroxy-24-oxo-5β-cholan-24-yl]amino]-ethane-sulphonic-acid;

(10) N-[3α,7β-dihydroxy-24-oxo-cholan-24-yl]amino-acetic acid;

(11) 3α,6α,12α-trihydroxy-5β-cholan-24-oic acid;

(12) 2-[[3α,6α,12α-trihydroxy-24-oxo-5β-cholan-24-yl-]amino]-ethane-sulphonic-acid;

(13) N-[3α,6α,12α-trihydroxy-24-oxo-cholan-24-yl]amino-acetic acid;

(14) 3α,6α-dihydroxy-5β-cholan-24-oic acid;

(15) 2-[[3α,6α-dihydroxy-24-oxo-5β-cholan-24-yl]amino]-ethane-sulphonic-acid;

(16) N-[3α,6α-dihydroxy-24-oxo-cholan-24-yl]amino-acetic acid;

(17) 3α,7α,12α-trihydroxy-23-hydroxy-5β-cholan-24-oic acid;

(18) 2-[[3α,7α,12α-trihydroxy-23-hydroxy-24-oxo-5β-cholan-24-yl-]amino]-ethane-sulphonic-acid;

(19) N-[3α,7α,12α-trihydroxy-23-hydroxy-24-oxo-cholan-24-yl]amino-acetic acid;

(20) 3α,7β,12α-trihydroxy-23-hydroxy-5β-cholan-24-oic acid;

(21) 2-[[3α,7β,12α-trihydroxy-23-hydroxy-24-oxo-5β-cholan-24-yl-]amino]-ethane-sulphonic-acid;

(22) N-[3α,7β,12α-trihydroxy-23-hydroxy-24-oxo-cholan-24-yl]amino-acetic acid;

(23) 3α,7α-dihydroxy-23-hydroxy-5β-cholan-24-oic acid;

(24) 2-[[3α,7α-dihydroxy-23-hydroxy-24-oxo-5β-cholan-24-yl-]amino]-ethane-sulphonic-acid;

(25) N-[3α,7α-dihydroxy-23-hydroxy-24-oxo-cholan-24-yl-]amino-acetic acid;

(26) 3α,7β-dihydroxy-23-hydroxy-5β-cholan-24-oic acid;

(27) 2-[[3α,7β-dihydroxy-23-hydroxy-24-oxo-5β-cholan-24-yl-]amino]-ethane-sulphonic-acid;

(28) N-[3α,7β-dihydroxy-23-hydroxy-24-oxo-cholan-24-yl-]amino-acetic acid; or from their pharmaceutically acceptable salts or complexes, said active principle being formulated with one or more excipient(s), which make it possible its release to be controlled over time, of the gradual-release, or repeated-action type, said active principle being contained in an amount of from 50 to 1200 mg, corresponding to the daily therapeutical dosage to be intaken even once daily.

According to the present invention, also all of the respective acceptable salts or complexes with various metals (e.g.: sodium) of the above identified compounds are intended as comprised.

As relates to the times of release of the controlled-release pharmaceutical compositions, the optimum solution would require the knowledge of the exact individual biorhythm of the stages of cholesterol metabolism, which are evidently subject to even wide fluctuations from subject to subject.

The unitary dosages to be intaken are comprised within the range of from 50 to 1,200 mg per intake unit; normally, one intake per day is foreseen, in the evening, before retiring. Only in particular cases two daily intakes of the base unit, in the morning and in the evening, shall be used.

As relates to the pharmaceutical techniques for producing the controlled-release preparations, reference is made to the treatises on this subject: e.g., Casadio.

However, we regard as suitable a brief description of the main release models, also for introductory purposes for the examples supplied in the following.

In order to simplify the discussion of the release models, defining the concept of "base unit" is useful: this is generally a single-dose pharmaceutical form for oral administration (capsule, tablet, pill, and so forth), containing the minimum therapeutical dosis, i.e., that dosis which is capable of rendering available an amount of active principle sufficient to cause the desired pharmacological response within time periods aligned with the properties of the drug, and determining its intrinsic availability for the absorption.

The models of controlled release of the active principle can be schematized as follows; however, it must be considered that, also in this case, other

technical solutions, not explicitly mentioned, but anyway technologically valuable and acceptable, must be considered at all analogous and absolutely non-limitative within the scope of the same invention:

1) Repeated-action model, with an action depending on the dissolving fluids: it supplies two or more base units, each one of which is available, at successive times, for the absorption within a portion of the alimentary canal characterized by particular chemical-physical conditions (pH, digestion enzymes, etc.); generally, a base unit, compatibly with its characteristics, is made immediately bioavailable for the absorption.

Distinguised are:

a) compact base unit: each base unit is coated as one portion only on all of its exposed surface; it makes it possible a good repeatibility to be obtained at the level of the desired sites, but can supply an even too quick repetition relatively to the variability of stomach emptying times; it is realized by coupling $n$ base units, all of them, or $n$-1 of them being protected with substances variously sensible to the particular properties of the dissolving medium: the coupling can be of parallel type (e.g., capsules containing 2 or more small tablets), or of serial type (e.g., concentric-sector tablets or pills, wherein the sequence of the base units must correspond to that of the dissolving fluids, or of the release sites);

b) subdivided base unit: each base unit is constituted by the set of a large number of sub-units, which become independent during the transit through the alimentary canal; due to probabilistic reasons, the variations in transit times are compensated for, and too near repetitions of the action are thus prevented, but the precision in release repeatibility decreases; it is realized by coupling $n$ base units, each constituted by a considerable number of sub-units, more or less integral with one another, and coating the different sub-units with substances variously sensible to the several dissolving media, homogeneously with one another within the same base unit (the coated base units can be all of them, or $n$-1 of them); the coupling of the various units can be of parallel type (e.g., gelatin capsules containing a mixture of chronoids) of serial type (e.g., concentric-sector tablets or pills, wherein the sequence of exposure to the release fluids and sites is respected), or of mixed type (e.g., mixed-granules tablets).

2) Repeated-action model, with an action independent from the dissolving fluids: it supplies 2 or more base units, each of which is available for the absorption at pre-established time intervals and substantially independent from the characteristics of the release environment; in general, the first base unit is immediately available, compatibly with the intrinsic characteristics; the definition of sub-classes is quite immaterial, because the theoretical model is bound to exclusively technological factors, whichever the pratical model, which one wants to realize, may be;

the model is realized by coupling base $n$ units, all of which, or $n$-1 of which, are variously protected drom the "environment" action by means of substances affected by factors which remain substantially identical throughout the alimentary canal; the coupling can be of parallel or serial type.

3) Long-lasting-or delayed-action model: this model supplies a base unit immediately available for the absorption and, subsequently, other fractions of the base unit, such as to maintain the action of the drug as constant as possible during a pre-established time period in addition to the customary individual dosis; the description of possible sub-classes seems to us of minor moment, in that the basic concept of any possible technological alternatives is to provide a rigorously repeatitive drug release curve, as independent as possible from the variations in the dissolving fluids; anyway, on a conceptual basis, three possible models can be defined:

a) homogeneously-subdivided model:

b) subdivided-release with negative gradient model:

c) compound-release model (immediate fraction + homogeneouly delayed fraction).

Even when the realized model reproduces the ideal curve of in vitro drug release (supposing that such an ideal curve can be determined), verifying in vivo different solutions seems anyway useful, in order to prevent possibilities of deviations between in vitro and in vivo tests; the model is realized by means of techniques allowing a gradual, reproducible,either quantitatively homogeneous or differentiated release of the active principle from the pharmaceutical form.

For a better understanding of the technical characteristics of the pharmaceutical compositions of the present invention, hereunder some examples are given, which relate to the above-reported compound No. 9, in its sodium salt form, and which are preceded by a short description of the adopted technology.

However, pharmaceutical compositions with other compounds of the above cited set, as well as their different acceptable salts, are to be considered at all homologous to the hereunder exemplified compositions, which therefore are not to be considered as limitative of the scope of the present invention.


Example 1

Repeated-Action Pill of 2 Base Units with Respectively Immediate Disintegration (Stomach) and Disintegration at pH> 7 (Distal Ileum, Colon)* Description of the pharmaceutical form: painted pill, with coated concentric sectors.

* Principle of practical model: use of known film-forming materials capable of forming coatings soluble at different pH values according to the used type.

* Elements constituting the pharmaceutical form:

Inner core: -Description:
Base unit destined to delayed disintegration
- Components:

    -Sodium 2-[[3α,7β-dihydroxy-24-oxo-5β-cholan-24-yl]-amino]-ethane-sulphonate 250 mg
    -Starch    15 mg
    -Microgranular cellulose    30 mg
    -CL Polyvinylpirrolidone    10 mg
    -Magnesium stearate    5 mg
-Adopted Technology:
Dry compacting process, followed by the pressing of the so-obtained granulate.

1st Layer: -Description:
Protective coating soluble at pH value higher than, or equal to, 7.
-Components:
    -Eudragit S 12.5P (dry substance)    40 mg
-Adopted Technology:
Spray process on rotary pill-making tray.

2nd Layer: -Description:
Immediate-disintegration base unit
-Components:

    -Sodium 2-[[3α,7β-dihydroxy-24-oxo-5β-cholan-24-yl]-amino]-ethane-sulphonate 250 mg
    -Polyvinylpirrolidone    50 mg
    -Polyethyleneglycol 6000    30 mg
    -Starch    40 mg
    -m.v.-Carboxymethylcellulose    20 mg
-Adopted Technology:
Spray process on rotary pill-making tray, with the components being fed to the process either dissolved or suspended with ethyl alcohol.

Peripheral Layer: -Description:
Finishing coating soluble at a pH value lower than 5.
-Components:

    -Eudragit E 12.5 (dry matter)    10 mg
    -Talc    5 mg
    -Titanium dioxide    2.5 mg
    -E 172 dye    0.02 mg
    -Alumina    0.08 mg
    -Polyethyleneglycol 6000    1 mg
-Adopted Technology:
The components are fed to the process either dissolved or suspended with the fluid paint Eudragit E 12.5.

* In vitro verification of the model.
The pills were tested in vitro by the method of USP, XIXth Edition, modified in the composition and in the sequence of the simulated gastrointestinal fluids;
the results were the following:
-After 1 hour at pH 1.5:
Peripheral layer and 2nd layer completely disintegrated, without any detectable modifications in the lower sectors;
-After 2-6 hours (pH 4.5-6.9):
Not any further disintegration;
-After 7 hours (pH 7.2):
Inner core completely disintegrated.

Example 2

Repeated-Action, Hard-Gelatin Capsule Containing 3 Base Units with Respectively Immediate Disintegration (Stomach), Disintegration at pH>6 (Proximal Ileum), and Disintegration at pH>7 (Distal Ileum, Colon)* Description of the pharmaceutical form: Hard-gelatin capsule, containing 3 tablets with different coating and colour.

* Principle of practical model: use of different known film-forming materials on each one of the three different base units (tablets) capable of forming coatings soluble at different pH values according to the used type.

* Elements constituting the pharmaceutical form:

1) Tablets: -Description:
Base unit destined to a subsequent differentiation - (immediate disintegration, or disintegration delayed by means of the different coatings, as hereunder reported).
-Components:

-Sodium 2-[[3α,7β-dihydroxy-24-oxo-5β-cholan-24-yl]-amino]-ethane-sulphonate
150 mg
-Starch    9 mg
-Microgranular cellulose    18 mg
-CL Polyvinylpirrolidone    6 mg
-Magnesium stearate    3 mg

-Adopted Technology:
Dry compacting process, followed by the pressing of the so-obtained granulate.

2 "A" Coating: -Description:
Protective coating soluble at pH value < = 5.

-Components:

-Eudragit S 12.5P (dry substance)    9 mg
-Talc    4.5 mg
-Titanium dioxide    2.25 mg
-Polyethyleneglycol 6000    0.9 mg

- Adopted Technology:
Spray process on rotary pill-making tray, on one third of the prepared tablets; the components are fed to the process either dissolved or suspended with the fluid paint Eudragit E 12.5.

3) "B" Coating: -Description:
Mixed-network protective film with a portion soluble at pH> = 6 + an insoluble portion.
-Components:

-Eudragit L 12.5P (dry matter)    18 mg
-Eudragit RS 12.5 (dry matter)    9 mg
-Talc    9 mg
-Titanium dioxide    4.5 mg
-E 172 dye    0.036 mg
-Alumina    0.144 mg
-Polyethyleneglycol 6000    1.8 mg

-Adopted Technology:
Spray process on rotary pill-making tray on one third of the prepared tablets; the components being fed to the process either dissolved or suspended with the mixture of the fluid paints Eudragit L 12.5P and RS 12.5.

4) "C"-Coating : -Description:
Mixed-network, protective coating, with a portion soluble at a pH> = 7 + an insoluble portion.
-Components:

-Eudragit S 12.5P (dry matter)    18 mg
-Eudragit RS 12.5 (dry matter)    9 mg
-Talc    9 mg
-Titanium dioxide    4.5 mg
-E 172 dye    0.108 mg
-Alumina    0.432 mg
-Polyethyleneglycol 6000    1.8 mg

-Adopted Technology:
Spray process on rotary pill-making tray on one third of the prepared tablets; the components being fed to the process either dissolved or suspended with the mixture of the fluid paints Eudragit S 12.5P and RS 12.5.

5) Coating constituted by capsules of hard gelatin.
-Description:
A container soluble in gastric juice, useful in order to gather the three tablets into one single intake dosage.

-Components: Hard gelatin; self-blocking capsules, size No. 00.
-Adopted technology:
Capsulation of three tablets, each one of which with coating of (A), (B), and (C), by automatic capsulation machine with tablet dispenser.
* In vitro verification of the model.
The pills were tested for disintegration in vitro by the method of USP, XIXth Edition, modified in the composition and in the sequence of the simulated gastrointestinal fluids; the results were the following:
-After 1 hour at pH 1.5:
Complete disintegration of the coating of hard gelatin and of the tablets with the (A) coating; no alterations observed on the tablets with the (B) and (C) coatings;
-After 2-3 hours (pH 4.5-6.9):
Not any further disintegration;
-After 4 hours (pH 6.9):
Complete disintegration of the tablets with (B) type coating;
- After 5-7 hours (pH 6.9-7.2):
Not any further disintegration;
-After 8 hours (pH 7.2):
Complete disintegration of all of the tablets, including those with a (C)-type coating.

In general, it was observed that the different formulations realized gave surprisingly satisfactory results, allowing a saving in active principle which resulted to be of the order of from 20 to 25%, with the therapeutical effect being the same. Furthermore, such formulations decrease the administering needs to one daily intake only (at maximum, to two daily intakes, the one in the morning and the other one in the evening), with self-explanatory advantages from the viewpoint of patient compliance in view of very long-lasting treatments.

In general, the initially stated purposes are efficaciously accomplished by the present invention.

## Claims

1. Pharmaceutical composition for oral usage, suitable for the management of the alterations of cholesterol metabolism, characterized in that it contains, as its active principle, a compound selected from the following compounds:

(1)  $3\alpha,7\alpha,12\alpha$-trihydroxy-$5\beta$-cholan-24-oic acid;

(2)  2-[[$3\alpha,7\alpha,12\alpha$-trihydroxy-24-oxo-$5\beta$-cholan-24-yl-]amino]-ethane-sulphonic-acid;

(3)  N-[$3\alpha,7\alpha,12\alpha$-trihydroxy-24-oxo-cholan-24-yl]amino-acetic acid;

(4)  $3\alpha,7\beta,12\alpha$-trihydroxy-$5\beta$-cholan-24-oic acid;

(5)  2-[[$3\alpha,7\beta,12\alpha$-trihydroxy-24-oxo-$5\beta$-cholan-24-yl-]amino]-ethane-sulphonic-acid;

(6)  N-[$3\alpha,7\beta,12\alpha$-trihydroxy-24-oxo-cholan-24-yl]amino-acetic acid;

(7)  2-[[$3\alpha,7\alpha$-dihydroxy-24-oxo-$5\beta$-cholan-24-yl]amino]-ethane-sulphonic-acid;

(8)  N-[$3\alpha,7\alpha$-dihydroxy-24-oxo-cholan-24-yl]-amino-acetic acid;

(9)  2-[[$3\alpha,7\beta$-dihydroxy-24-oxo-$5\beta$-cholan-24-yl]amino]-ethane-sulphonic-acid;

(10)  N-[$3\alpha,7\beta$-dihydroxy-24-oxo-cholan-24-yl]amino-acetic acid;

(11)  $3\alpha,6\alpha12\alpha$-trihydroxy-$5\beta$-cholan-24-oic acid;

(12)  2-[[$3\alpha,6\alpha,12\alpha$-trihydroxy-24-oxo-$5\beta$-cholan-24-yl-]amino]-ethane-sulphonic-acid;

(13)  N-[$3\alpha,6\alpha,12\alpha$-trihydroxy-24-oxo-cholan-24-yl]amino-acetic acid;

(14) $3\alpha,6\alpha$-dihydroxy-$5\beta$-cholan-24-oic acid;

(15)  2-[[$3\alpha,6\alpha$-dihydroxy-24-oxo-$5\beta$-cholan-24-yl]amino]-ethane-sulphonic-acid;

(16)  N-[$3\alpha,6\alpha$-dihydroxy-24-oxo-cholan-24-yl]amino-acetic acid;

(17)  $3\alpha,7\alpha,12\alpha$-trihydroxy-23-hydroxy-$5\beta$-cholan-24-oic acid;

(18)  2-[[$3\alpha,7\alpha,12\alpha$-trihydroxy-23-hydroxy-24-oxo-$5\beta$-cholan-24-yl-]amino]-ethane-sulphonic-acid;

(19)  N-[$3\alpha,7\alpha,12\alpha$-trihydroxy-23-hydroxy-24-oxo-cholan-24-yl]amino-acetic acid;

(20)  $3\alpha,7\beta,12\alpha$-trihydroxy-23-hydroxy-$5\beta$-cholan-24-oic acid;

(21)  2-[[$3\alpha,7\beta,12\alpha$-trihydroxy-23-hydroxy-24-oxo-$5\beta$-cholan-24-yl-]amino]-ethane-sulphonic-acid;

(22)  N-[$3\alpha,7\beta,12\alpha$-trihydroxy-23-hydroxy-24-oxo-cholan-24-yl]amino-acetic acid;

(23)  $3\alpha,7\alpha$-dihydroxy-23-hydroxy-$5\beta$-cholan-24-oic acid;

(24)  2-[[$3\alpha,7\alpha$-dihydroxy-23-hydroxy-24-oxo-$5\beta$-cholan-24-yl-]amino]-ethane-sulphonic-acid;

(25)  N-[$3\alpha,7\alpha$-dihydroxy-23-hydroxy-24-oxo-cholan-24-yl-]amino-acetic acid;

(26)  $3\alpha,7\beta$-dihydroxy-23-hydroxy-$5\beta$-cholan-24-oic acid;

(27)  2-[[$3\alpha,7\beta$-dihydroxy-23-hydroxy-24-oxo-$5\beta$-cholan-24-yl-]amino]-ethane-sulphonic-acid;

(28)  N-[$3\alpha,7\beta$-dihydroxy-23-hydroxy-24-oxo-cholan-24-yl-]amino-acetic acid;

or from their pharmaceutically acceptable salts or complexes, said active principle being formulated with one or more excipient(s), which make it possible its release to be controlled over time, of the gradual-release, or repeated-action type, said active principle being contained in an amount of from 50 to 1200 mg, corresponding to the daily therapeutical dosage to be intaken even once daily.

2. Composition according to claim 1, characterized in that it is in the form of a single daily intake unit.

3. Composition according to claim 1, characterized in that it is in the form of two daily intake units, equivalent to each other.

4. Composition according to claim 1, characterized in that said release controlled over time is of the gradual-release, or long-lasting-action type.

5. Composition according to claim 1, characterized in that said release controlled over time is of the repeated-action type.

6. Composition according to as hereinabove disclosed, in particular as disclosed in the Examples.

## European Patent Office

# EUROPEAN SEARCH REPORT

Application Number

EP 87 20 2214

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 106, no. 9, 2nd March 1987, page 45, abstract no. 61052x, Columbus, Ohio, US; M. MASUDA et al.: "Effect of taurine and homotaurine on bile acid metabolism in dietary hyperlipidemic rats" & J. PHARMACOBIO-DYN. 1986, 9(11), 934-40 * Whole abstract * --- | 1-6 | A 61 K 31/575 A 61 K 9/22 |
| X,Y | US-A-3 859 437 (A.H. WEIGAND) * Column 1, lines 9-19; column 2, lines 18-45; column 3, line 65 - column 4, line 6; column 4, lines 18-34; example 1; claims * --- | 1-6 | |
| X,Y | FR-A-2 518 880 (PROTER SPA) * Claims; page 5, lines 25-29 * --- | 1-6 | |
| X,Y | GB-A-2 036 558 (LEHNER AG) * Example 1a; claims * ----- | 1-6 | TECHNICAL FIELDS SEARCHED (Int. Cl.4) A 61 K C 07 J |

The present search report has been drawn up for all claims

| Place of search | Date of completion of the search | Examiner |
|---|---|---|
| THE HAGUE | 29-02-1988 | BERTE M.J. |

EPO FORM 1503 03.82 (P0401)